# EUROPEAN PATENT APPLICATION

(11) **EP 4 082 353 A1**
(43) Date of publication of application: **02.11.2022**
(21) Application number: 20906409.6
(22) Date of filing: 10.12.2020
(51) Int. Cl.: A23J 1/08, A23J 1/20, A23L 33/17

(54) **HYPERPROTEIC SNACK-TYPE FOOD PRODUCT WITH HIGH BIOLOGICAL VALUE (HBV) FOR PATIENTS UNDERGOING HAEMODIALYSIS**

(30) Priority: 24.12.2019 CL 20193831
(71) Applicant: Universidad de Antofagasta, Antofagasta (CL)
(72) Inventor: VEGA SAAVEDRA, Marcela, Antofagasta (CL)
(74) Representative: Pons
(86) International application number: PCT/CL2020/050171
(87) International publication number: WO 2021/127796

(57) **Abstract**

The present snack-type food product with high hypoproteic content comprises proteins of high biological value (HBV), in which the principal macronutrients are protein, sugars, carbohydrates and fats. This snack-type food product was specifically developed for patients undergoing haemodialysis treatment. The snack-type food product has sensory characteristics that allow it to achieve better acceptance in comparison with other products available on the market

## Description

### FIELD OF THE INVENTION

The present invention refers to a high protein or hyperproteic snack type food product, comprising proteins of high biological value for patients undergoing hemodialysis. The food product is selected from a cookie, cake, individual portion, among others.

### BACKGROUND

The pathological processes that affect humans are diverse and complex, producing extremely important metabolic alterations, mainly nutritional, such as Chronic Renal Insufficiency (CRF). CKD corresponds to the clinical situation derived from permanent and progressive loss of kidney function that can be reached by multiple etiologies, both congenital and/or hereditary as well as acquired. In its terminal stage (ESRD), it constitutes a global public health problem, with an increasing incidence and prevalence, poor prognosis and high cost. It requires renal replacement treatment by dialysis or kidney transplantation. The causes vary from country to country.

This pathology is a serious health problem that significantly impacts affected people, their families, society and health services. It is frequently associated with other chronic diseases such as diabetes mellitus (DM), high blood pressure (HTN), and heart disease. In addition to putting the person at risk for ESRD, it increases the risk of cardiovascular morbidity and mortality, at levels 10 times that of the average risk in the population. A direct and independent association has been demonstrated between the deterioration of renal function and an increased risk of events and death due to cardiovascular diseases and hospitalization rate (Ministry of Health; Government of Chile. (2010). Clinical guide for prevention of chronic kidney disease. Retrieved on November 29, 2013, from MINSAL: http://web.minsal.cl/portal/url/item/955578f79a1bef2ae04001011f01678a.pdf).

The classification of CRF (Table 1) is based exclusively on its intensity, traditionally establishing five levels based on glomerular filtration (GFR). (Mataix Verdú, 2009).

| Table 1: IRC Classification IRC | | |
|---|---|---|
| Stadium | Description | FG (ml/m) |
| I | FG normal, kidney damage | >90 |
| II | IRC low | 60-89 |
| III | IRC moderate | 30-59 |
| IV | IRC severe | 15-29 |
| V | IRC terminal | <15 |

Maintaining adequate nutrition when suffering from ESRD can help delay the deterioration of kidney function and avoid complications due to water and electrolyte imbalance and protein-calorie malnutrition. Protein-caloric malnutrition is a frequent manifestation in patients with ESRD. Protein catabolism increases due to impaired glucose metabolism. The use of proteins to generate energy jeopardizes normal protein function in terms of maintaining cell structure, repairing tissues, regulating the pH of the blood, and forming antibodies to resist infection. Increasing dietary protein intake can lead to increased accumulation of metabolites in the blood. Nutritional treatment for people with ESRD should include adequate amounts of fats and carbohydrates to limit protein catabolism and waste accumulation (Schub, E., Schub, T., & Pravikoff, D. (June 29, 2012). Nursing up to date. Retrieved on June 17, 2014, from Chronic renal failure: nutritional treatment: http://web.b.ebscohost.com/nrc/detail?vid=2&sid=c66ed342-19c7-4e17-adea-e432908d944b% 40sessionmgr1 10 & hid = 126 & bdata = Jmxhbmc9ZXMmc2IOZT 1 ucm Mtc3Bh # db = nre & AN = SPA5000004692).

Among the important factors to know the progression of the damage are glomerular filtration rate (GFR), blood urea nitrogen (NUS), control of proteinuria, among other tests. When the GFR is less than 15 ml/min, the patient requires dialysis, which is a process in which water and solutes are exchanged bidirectionally between two solutions of different composition, which are separated from each other by a semi-permeable membrane. This membrane allows the passage of water and molecules of small and medium molecular weight but prevents the passage of albumin (ALB), molecules of greater weight and cells. The dialysis process is also a clear indication for patients with severe symptomatic uremias, uncontrollable hyperkalemia, severe metabolic acidosis and volume overload with acute lung edema and/or refractory arterial hypertension (Mataix Verdú, J. (2009). Kidney diseases; Renal physiological bases. In J. Mataix Verdú, Treaty of Nutrition).

The most widely used method to replace kidney function is Phlemodialysis (FID), this is performed every three weeks (4-5 hours at a time) through a catheter placed in a major vein (jugular, femoral) or through a fistula arteriovenous (AVF, internal ligation of an artery with a vein) generally constructed at the level of the upper limb (P. Rodota, L., & Castro, ME (2012). Clinical nutrition and Diet therapy. Buenos Aires, Argentina: Editorial Medica Panamericana). IDF is an extracorporeal blood clearance technique that partially supplies the renal functions of excreting water and solutes and regulating acid-base and electrolyte balance. It does not supply endocrine or renal metabolic functions. It consists of interposing a semi-permeable membrane between two liquid compartments (blood and dialysis fluid). For this, a filter or dialyzer is used. The semi-permeable membrane allows circulating water and small and medium molecular weight (MW) solutes, but not high molecular weight proteins or blood cells, too large to pass through the pores of the membrane. There are two physical mechanisms that regulate these functions: diffusion or transport by conduction and ultrafiltration or transport by convection (Sellarás, VL, Torres Ramírez, A., Hernández Marrero, D., & Ayus, JC (nd). Manual de Nephrology. Harcourt).

HD has quite a few complications in the patient. Determinants of the morbidity and mortality of this process are the NE and the adequacy of dialysis (Kt/v, a measure of the total clearance of urea normalized or corrected for its volume of distribution, is a good measure of the dialysis dose, of its intensity or the effect of dialysis, its intensity or the effect of the patient's dialysis, Vega Saavedra, M. (2012). Nutrition in Chronic Renal Insufficiency. Santiago, Chile), which is interrelated when observing that the Correctly dialysed patients present greater general well-being and therefore better intake (P. Rodota, L., & Castro, ME (2012). Clinical nutrition and Diet therapy. Buenos Aires, Argentina: Editorial Medica Panamericana).

Other complications are, for example, protein-calorie malnutrition and inflammation, possible causes include poor food intake, hormonal and gastrointestinal disorders, rigorous dietary restrictions, use of medications that can influence the absorption of nutrients, insufficient dialysis, constant presence of intercurrent diseases, oxidative stress, loss of nutrients through HD, anorexia, metabolic acidosis, uremia, decreased clearance of inflammatory cytokines, depending on the degree of severity is usually associated with poor prognosis, decreasing quality of life and increasing hospitalization time and mortality (Young, P., Lombi, F., Finn, B., Forrester, M., Compolo Girard, V., Pomeranz, V., and others. (2011). Complex syndrome of malnutrition and inflammation in chronic hemodialysis. Retrieved on December 3, 2013, from Medicina, Buenos Aires: hltp: //www.nefrohospbritanico.org.ar/pdfs/MICS2011 .pdf). This is justified, since the hemodialysis procedure is itself hypercatabolic, increasing muscle proteolysis and loss of nutrients in the dialysate (Miguel C. Riella; Cristina Martins. (2004). Nutrition and Kidney. Brazil: Editorial Medica Panamericana), the type of dialytic membrane affects the protein metabolism of HD patients. The interaction between blood and regenerated cellulose membranes leads to accelerated protein splitting (post-dialytic catabolism). Complement and leukocyte activation induced by bioincompatible dialysate membranes can lead to the release of proinflammatory cytokines and cause muscle breakdown with AA release. There is a significant increase in infection-related deaths in patients treated with bioincompatible dialysis membranes. It is possible that a deficient NE increases the prevalence of infection in these patients and, sometimes, also the risk of death (Miguel C. Riella; Cristina Martins. (2004). Nutrition and Kidney. Brazil: Editorial Medica Panamericana). One of the peculiarities of ESRD in dialysis is malnutrition in patients, which is an important reversible factor that contributes to mortality in this population. (Miguel C. Riella; Cristina Martins, 2004). Worldwide, the market has designed supplements in order to counteract protein-caloric malnutrition in patients with this pathology, however, they are expensive, have a high percentage of humidity, little variety of products and in some they are not restricted the patient's special micronutrient needs.

The dialysis procedure as such is energy demanding and constitutes a protein loss of about 10 grams for each procedure, this is supported by studies carried out in hemodialysis (HD) patients, where the loss of linked amino acids (AA) was analyzed. and free, with a range of 9 to 13 grams, which gives an average total loss of 10 grams of proteins per dialysis session (Miguel C. Riella; Cristina Martins. (2004). Nutrition and Kidney. Brazil: Editorial Medica Panamericana) Furthermore, if we consider the lack of appetite inherent to this type of patient, demonstrated by: rejection of meat consumption, insufficient food intake, among other factors; causes this patient to present mainly protein-calorie malnutrition (KDOQUI. (2000). American Journal of Kidney Diseases. The Official Journal of the National Kidney Foundation, 35 (6)).

On the other hand, hypoalbuminemia in these patients may be due to factors unrelated to nutrition such as overhydration or inflammation. The poor results obtained with the administration of caloric-protein supplements in hypoalbuminaemic patients on HD also suggest that hypoalbuminemia is not always a marker of malnutrition. The best predictor of serum ALB levels is CRP, which, in turn, predicts mortality better than ALB. Higher levels of CRP are detected in ESRD than in the general population, with these levels being higher in HD patients, which has led to speculation about the possibility that the HD process may cause a certain degree of inflammation. In this sense, it has been seen that during HD with less biocompatible membranes there is a certain degree of chronic inflammatory response and a decrease in the levels of certain serum nutrition markers (Fernandez Reyes, M., Alvarez Unda, F., Sanchez Mon, R., Iglesias, P., & Vásquez, A. (2000). Nutritional status, comorbidity and inflammation in hemodialysis. Retrieved on November 28, 2013, from the Nephrology Service and Endocrinology Service of the General Hospital of Segovia: http: //www.revistanefrologia.com/revistas/P1 -E45/P1 -E45-S1829-A10248.pdf). As a result of malnutrition due to deficit, it is necessary to carry out an evaluation of the EN, considering anthropometry such as the measurement of weight, height, arm circumference, skin folds (biceps, triceps, subscapularis and supra iliac). From the dry weight (without the presence of edema, hypertension or another sign of fluid overload. That is, when the dialysate completes the procedure without metabolic alterations) and the height (it is important to regularly assess the dry weight), the index is calculated body mass (BMI); arm circumference is indicator of skeletal muscle mass, and skin folds are indicators of fat body mass, measurements should be made in the arm that does not have AVF (P. Rodota, L, & Castro, ME (2012). Clinical Nutrition and Diet Therapy Buenos Aires, Argentina: Editorial Medica Panamericana).

Due to marked anorexia, these patients are unable to voluntarily ingest caloric and protein requirements, since the caloric goal to be achieved is 35 calories/kg of dry weight/day and a protein intake greater than or equal to 1.2 g/kg of dry weight/day, with a minimum of 60% to 70% of AVB proteins. If the protein-calorie needs cannot be met with the normal diet, oral nutritional supplements or enteral supplementation can be used either by nasogastric or nasojejunal tube with infusion pumps, including parenteral nutrition during HD. All these supplements must respect the adequate fluid volumes (500 ml/day plus residual diuresis), in addition to the amount of critical micronutrients such as sodium (1 to 3 g/day and in case of anuria 2 g/day), phosphorus (800 to 1000 mg/day) and potassium (1 to 3 g/day) (P. Rodota, L, & Castro, ME (2012). Clinical nutrition and Diet therapy. Buenos Aires, Argentina: Editorial Medica Panamericana).

Food supplements are expensive, also due to the prolonged time of use and the limited variety of them, the patient abandons their consumption or simply rejects them, this means a greater protein-caloric debt, which transforms into a decrease in muscle mass, weight loss, decreased ALB, among others (Huerta Loza, E. (2007). Nutritional aspects in dialysis. Retrieved on December 3, 2013, from Hospital S. Milan, Nephrology Section: http://www.euskomedia.org/PDFAnlt/osasunaz/08/08139149.pdf)) . Among the patent documents can be mentioned US6346284B1 (Nutriset; Inszt Rech Dedvlopment IRD) refers to low osmolality energy rich foods containing at most 10 % of its weight in water. Food can provide all the nutrients and, in particular, minerals and all the vitamins necessary for the restoration of malnourished patients. The food supplement can be used in diets in which cereal is an essential part of the food intake. The food is insoluble in water before dispersion by agitation and does not generate hyperosmolality and is stable to oxidation.

US4183966A (Univ. Oklahoma State) refers to a method of making a high protein snack that includes the steps to inoculate the whey, as a byproduct of cheese making, with yeast, incubate the mixture with aeration until the yeast use virtually all of the whey lactose, heating the culture to precipitate the whey protein, which separates the whey protein and yeast cells to provide a paste-like material, mixing the whey protein and the paste of yeast cells with a filling consisting of potato flakes or cornstarch or a mixture of the two, plus baking powder or baking soda (to reduce acidity), plus salt and egg whites, in a dough, extrude the dough to provide sandwich-like pieces, fry the pieces in a deep fryer and then cook the fried pieces in a microwave oven.

US3814819A (Pillsbury CO) refers to a protein fortified food bar of a controlled caloric content composed of stacked crunchy wafers with creamy filling, where each wafer is composed of 10 parts of flour, 6 parts of protein for example sodium caseinate or a Lactoalbumin-casein precipitate, 0.8 parts oil as release agent and small amounts of chemical yeast, the filling consisting of 10 parts of shortening, 10 parts of finely divided protein such as milk protein, 6 parts of sugar, a minor amount of flavoring, if desired, and a vitamin and mineral mixture, if desired, and confectionery coatings can be applied on the stick, if desired. US3480442A (Archer Daniels Midland CO) refers to a process for the preparation of an edible, crunchy, friable snack, where the cells have a random distribution and size of high protein content prepared by extrusion of a mixture of proteins from a protein derivative solid having a protein content of at least 30% by weight of the solid and from 12 to 20% by weight of the water mixture at a temperature of 200 to 480 ° F, at a pressure of at least 1000 psi.

CA2691691C (Baum Seth J) which refers to nutritional compositions and methods of preparation, useful in the treatment of kidney diseases, where the compositions comprise vitamins, minerals, amino acids and/or proteins in amounts that can be used to supplement the nutritional deficiencies observed in patients with kidney disease, kidney failure, or end-stage renal disease. The compositions can also be used as nutritional supplements for patients undergoing dialysis therapy or for patients on a restricted diet. Furthermore, the compositions can be used in combination or alone as a method of treating or managing a subject in various stages of chronic kidney disease, or during disease progression.

EP0747395B1 (Clintec Nutrition CO) an enteral composition to provide nutrition to kidney patients. The enteral composition includes an effective amount of a protein source that includes whey protein and free amino acids that provide essential and non-essential amino acids. The composition is calorie-dense and has a moderate osmolality.

Therefore, it is necessary to prepare a food product that contains the characteristics of a snack-type food product in order for the patient to partially recover the proteins that he has lost during the HD process. Given the protein wasting, the chronic inflammatory state, the deficit of protein intake and the poor adherence to traditional nutritional supplements, the present invention provides a very useful snack-type food product for an average patient on HD (See Table 2) containing protein from AVB, which is sensory pleasing, moderate in cost and low in humidity (avoids excess fluid consumption).

Annex 2: Requirements of an average patient undergoing the HD process.

| Macromolecule | Request |
|---|---|
| Calories | 35 calories/kg dry weight/day |
| Proteins | 1.2 grams/kg dry weight/day |
| Lipids | 1 grams/ kg dry weight/day |
| HC | 4.5 grams/ kg dry weight/day |
| Phosphorus | 800 - 1000 mg/day |
| Potasium | 1-3 g/day |

| | |
|---|---|
| Sodium | 1 -3 g/day |
| Water | 500 ml/day + urinary Volume 24 hours |

To favor its conservation and storage, specific food additives are used, which do not negatively affect the consumption of critical micronutrients in the patient on HD (see table 3), and thus favor the consumption of the product (Fernandez Reyes, M., Alvarez Unda, F., Sanchez Mon, R., Iglesias, P., & Vásquez, A. (2000). Nutritional status, comorbidity and inflammation in hemodialysis. Retrieved on November 28, 2013, from the Nephrology Service and the Endocrinology Service of the Hospital General de Segovia: http://www.revistanefrologia.com/revistas/P1- E45/P1 -E45-S1829-A10248.pdf).

**Table 3: Food additives.**

| | |
|---|---|
| • Wetting-stabilizing substances | Sorbitol: Hygroscopic solid used in the food industry as moisturizer to maintain several products with an appropriate moisture grade. |
| • Antioxidants | BHA: used to protect fats used in confectionery industry. |
| • Leavening agent | Disodium diphosphate: Used in the food industry as leavening agent, moisturizer, acid regulator, emulsifying and sequestrant agent. |
| • Preservative agent | Propionic acid: Used as preservative agent, inhibiting mold and some bacteria growth (appropriated dose: 1 gr/kg) |

To obtain the product with the desired characteristics, technology from the food industry was used, complementing its quality and acceptance, through sensory and proximal analysis.

### BRIEF SUMMARY OF THE INVENTION

In the market there are food products focused on patients undergoing hemodialysis treatment, that is, products high in protein content. However, patients they have shown a poor tolerance to them due to the sensory and physical characteristics.

On the other hand, it must be taken into consideration that a hypoprotein liquid formula is not suitable in patients should limit the consumption of liquids. A patient is on dialysis, likely urinates little or no urine, and any extra fluid must be removed through dialysis. Therefore, excessive fluid consumption can cause accumulation between dialysis sessions, which will mainly cause the following symptoms: headaches and low energy, swelling in your face, hands and feet (edema), difficulty breathing from the liquid in the lungs, heart damage from forcing the heart with too much fluid, and high blood pressure that can lead to stroke.

For the development of a snack-type food product with characteristics similar to an aerated dessert, it will also be transformed into a liquid, showing the same disadvantages as the liquid formulations mentioned above.

The present invention then provides a solid snack-type product with a high protein content, that is, hypoprotein, with proteins of high biological value (BVS) that comprises a mixture of components that satisfies the requirements of patients undergoing dialysis, and that was developed considering mainly the following aspects: the nutritional requirements of patients undergoing hemodialysis treatment; the contribution of the mixture of raw materials; the selection of the most suitable protein; the identification of the most suitable preparation, different mixtures were made to identify the best mixture of the products; the improvement of the formulation, adding fiber that helps regulate phosphorus levels in the blood and natural antioxidant products that have an anti-inflammatory effect.

The present snack-type food product with a high hypoprotein content comprises proteins of high biological value (BVS), the main macronutrients being protein, carbohydrates, carbohydrates and fats. This snack type food product was developed specifically for patients undergoing hemodialysis treatment. This snack-type food product has sensory characteristics that allow it to reach a better acceptance, compared to other existing products on the market.

### BRIEF DESCRIPTION OF THE FIGURES

Figure 1 shows the percentage preference in terms of taste of the Snack 1.1 sample (ovalbumin sugar free). 46% "Like", and 4% "Dislike Too Much."
Figure 2 shows the preference according to Flavor over the Snack 1.2 sample (ovalbumin with sugar), 27% "Indifferent", 27% too disliked ", and 4%" Too much disliked ".
Figure 3 shows the Preference according to Flavor over the sample of Snack 2.1 (Lactalbumin sugar free), 34% "Disliked too much", and 4% "He likes it".
Figure 4 shows the preference according to flavor of the sample of Snack 2.2 (Lactalbumin with sugar), 54% "I Like Too Much", and 0% "I dislike Too Much".
Figure 5 shows the preference according to taste of sample 3.1 (spirulina + lactalbumin with sugar), 42% "Too Dislike", and 8% "Too Much Dislike".
Figure 6 shows the preference according to Flavor to the sample of Snack 3.2 (spirulina + sugar-free lactalbumin), 35% "Too Unpleasant", and 4% "Too Much Dislike".
Figure 7 shows the preference according to Texture to the sample of Snack 1.1 (ovalbumin sugar free), 31% "I Dislike", and 11% "Too Dislike".
Figure 8 shows the preference according to Texture of the Snack 1.2 sample (ovalbumin with sugar), 31% "Too Dislike", and 4% "Too Much Dislike".
Figure 9 shows the preference according to the texture of the Snack 2.1 sample (sugar-free lactalbumin), 27% "Disliked Too Much", 27% "Disliked", and 12% "Disliked Too Much".
Figure 10 shows the preference according to Texture of the Snack 2.2 sample (lactalbumin with sugar), 46% "Like" and 11% "Dislike the texture".
Figure 11 shows the preference according to Snack Texture 3.1 (spirulina + lactalbumin with sugar), 38% "Pleasant" and 4% "Too Much Pleasant".
Figure 12 shows the preference according to Texture of sample 3.2 (spirulina + sugar-free lactalbumin), opinions very varied. 23% "Dislike Too Much", 23% "Dislike", "Indifferent" and "Pleased" and 8% "Dislike Too Much".

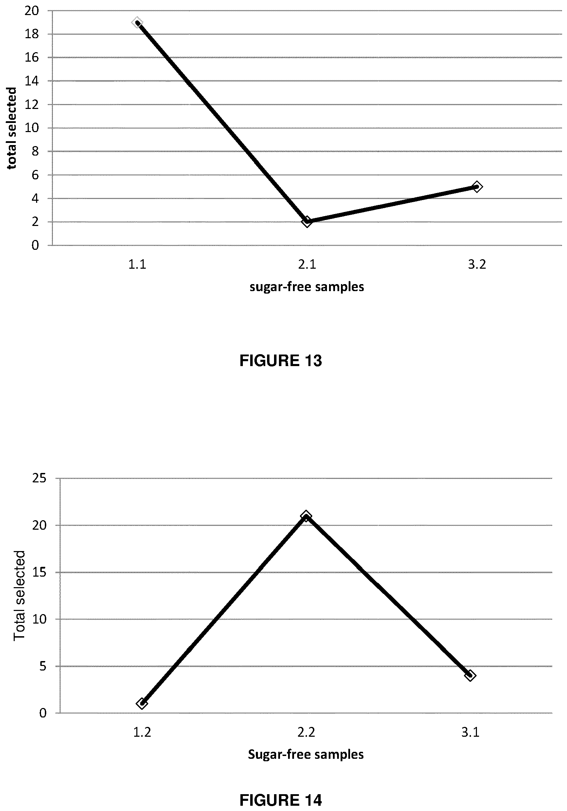

Figure 13 shows that the snack sugar free (ovalbumin sugar free) was the most preferred according to Flavor and Texture, while the least preferred was the sample Snack 2.1 (lactalbumin sugar free).
Figure 14 shows that the snack with sugar (lactalbumin with sugar) was the most preferred according to Flavor and Texture, while the least preferred was the sample of Snack 1.2 (ovalbumin with sugar).

### DESCRIPTION OF THE INVENTION

The present invention refers to a "snack" type light food product for consumption between meals that comprises three types of proteins of high biological value (BVS), that is, grams of proteins that can replace the grams of body proteins of an adult person/100 grams of dietary protein (Spreer, D. (1991). Industrial Lactology. In DI Spreer, Milk, preparation and processing, machines, installations and equipment. Dairy products (page 20). Germany: ACRIBIA, SA), useful to improve nutritional status (NE) in patients with ESRD on hemodialysis (HD). The product of the invention is inexpensive, with characteristics that allow adequate acceptability, low in humidity, easy to preserve and durability, and competitive with existing products on the market for patients with IRCTHD.

The present solid snack food product comprising: proteins and at least one or more sweetening agents, leavening agent, binding agents, binding agents, flavoring agents, flavoring agents, and optionally preservative agents, confectionery coating agents, among other agents used. in food preparation. Preferably, said proteins are proteins of animal or natural origin. Even more preferably, said proteins are proteins of animal origin selected from lactoalbumin or ovalbumin. More preferably still, said protein of animal origin is ovalbumin.

Preferably, said binding agent is flour.

Preferably, said lipid emulsifying agent is margarine.

Preferably, said binding agent is dehydrated egg white, egg or both. Preferably, said raising agent is baking powder.

Preferably, said flavoring and flavoring agent is coconut or vanilla essence. Preferably, said sweetening agent is selected from sugar or stevia.

Preferably, the ratio of binding agent to lipid emulsifying agent to binding agent to leavening agent to sweetening agent is 150: 150: 90: 10: 10: 31.2.

The protein sources of the light snack-type food product are selected due to their characteristics, such as digestibility (an important factor in determining the nutritional value of a protein, this is calculated by the ratio of nitrogen ingested in the diet and nitrogen eliminated in the feces , expressed as a percentage Proteins of animal origin have a high digestibility above 95%, whereas those of vegetable origin have values lower than 80%), availability in the market, biological value, etc. Such protein sources are listed below:
1. Proteins of Animal Origin - Lactalbumin (ALB): This protein is found in whey and the proportion of ALB is approximately 16 to 18%. This protein does not contain any or almost no phosphorus, contrary to what happens with casein, which although it is also a component of whey, is a phosphoprotein because it has strongly bound phosphate groups and also establishes links with the calcium. Whey proteins have a great physiological and nutritional value, since their biological value (BV) is 124 (Spreer, D. (1991). Industrial Lactology. In DI Spreer, Milk, preparation and processing, machines, installations and devices Dairy products (page 20). Germany: ACRIBIA, SA). Preferably, lactoalbumin corresponds to a whey protein powder supplement for patients with an increased need for protein. The amount of protein in 100 g of Protein Source product used is 87 grams.
2. Protein of Plant Origin - Spirulina: It is one of the oldest plants on earth, it is generated naturally in the oceans, the protein content in this algae is 65% higher than that of any other natural food. In spirulina all essential amino acids (phenylalanine, isoleucine, leucine, lysine, methionine, threonine, tryptophan, valine, arginine and histidine) and non-essential (alanine, tyrosine, aspartate, cysteine, glutamate, glutamine, glycine, proline, serine, asparagine and arginine) known. It also has the advantage that its proteins are easily digested and assimilated by the human body (LJ (November 2002). D Salud Discovery. Retrieved on November 6, 2014, from Spirulina Super Food of the Future: http://www .dsalud.com/index. php? page = article & c = 798). The amount of protein in 100 g of the Protein Source product used is 65 grams (Morales Fernandez, C. (April 2011). Loving Life. Retrieved on November 10, 2014, from Organic Spirulina Powder: http://lovinglife.bootic.net/products/espirulina-organica-en-polvo-150) 3. Protein of Animal Origin - Ovalbumin: This protein is equivalent to 60 % of total protein from egg white. It is possible to find each and every one of the essential amino acids (phenylalanine, isoleucine, leucine, lysine, methionine, threonine, tryptophan, valine, arginine and histidine) in adequate quantities to meet the nutritional needs of individuals, presenting a digestibility of 97 % (Suarez Lopez, M., Kizlanski, A., & Lopez, L. (2006). Evaluation of the quality of proteins in foods by calculating the amino acid score corrected for digestibility. Hospital Nutrition, 0212-1611). Preferably, the ovalbumin is dehydrated egg white. The amount of protein in 100 g of Protein Source product used is 74 grams.

These 3 protein sources were used to ensure the variety of the food product, ensuring an average protein intake (15 gr per product), evaluating acceptability. Table 4 shows the coding of each sample of food product for the experimental tests, and thus differentiate what type of protein source was used and its sugar-free or sugar-free variety.

| Table N°4: Coding of hyperproteic food products and its variants | |
|---|---|
| Code | Type of protein and variant |
| 1.1 | Ovoalbumin sugar free |
| 1.2 | Ovoalbumin with sugar |
| 2.1 | Lactoalbumin sugar free |
| 2.2 | Lactoalbumin with sugar |
| 3.1 | Spirulina + lactoalbumin with sugar |
| 3.2 | Spirulina + lactoalbumina sugar free |

A recipe was designed for each type of protein source, with 2 varieties (with sugar and sugar free) these are: Snack of ovalbumin with sugar, Snack of ovalbumin sugar free, Snack of lactalbumin with sugar, Snack of lactalbumin sugar free, Snack of Spirulina with sugar, Spirulina snack sugar free (see Table 5). Each variety of snack was standardized with: Calories: 350-400 kcals. Proteins: 17 gr/serving (VCT> 15%). Lipids: 10-12 gr/portion (VCT > 25%). Carbohydrates: 45-50 gr/serving (VCT> 50%), where VCT means Total caloric value, that is, the amount of calories necessary to replace the heat lost by the body, and which is provided by all the food eaten daily.

**Table 5: Basic Recipe Book proposed for the creation of the Hyperproteic Snack.**

| | | | |
|---|---|---|---|
| Hyperproteic Snack with dehydrated egg white with sugar | | Hyperproteic Snack with dehydrated egg white sugar free | |
| Ingredients: | | Ingredients: | |
| | • Flour as a binding agent 150 grs | | • Flour as a binding agent 150 grs |
| | • Sugar as sweetening agent 125 grs. | | • Margarine as lipidic emulsifying agent 150 gr. |
| | • Margarine as lipidic emulsifying agent 100 gr. | | |
| | | | • Dehydrated egg white as binding agent 90 gr. |
| | • Dehydrated egg white as binding agent 90 gr. | | |
| | | | • Eggs as binding agent 2 un. |
| | • Eggs as binding agent 2 un. | | • Baking powder as leavening agent 10 gr. |
| | • Baking powder as leavening agent 10 gr. | | |
| | | | • Coconut as flavoring and aromatizing agent 10 gr. |
| | • Coconut as flavoring and aromatizing agent 10 gr. | | |
| | | | • Vanilla essence as a flavoring and aromatizing agent 5 ml. |
| | • Vanilla essence as a flavoring and aromatizing agent 5 ml. | | |
| | | | • Stevia as sweetening agent 31.2 ml |
| Hyperproteic Snack: Whey protein powder supplement with sugar | | Hyperproteic: Whey protein powder supplement sugar free | |
| Ingredients: | | Ingredients: | |
| | • Flour as a binding agent 150 grs | | • Flour as a binding agent 150 grs |
| | • Sugar as sweetening agent 125 grs. | | • Margarine as lipidic emulsifying agent 150 gr. |
| | • Margarine as lipidic emulsifying agent 100 gr. | | |
| | | | • Whey protein powder supplement sugar free as protein source 80 gr. |
| | • Whey protein powder supplement with sugar as protein source 80 gr. | | |
| | | | • Huevos como agente ligante 2 un. |
| | • Eggs as binding agent 2 un. | | • Baking powder as leavening agent 10 gr. |
| | • Baking powder as leavening agent 10 gr. | | |
| | • Coconut as flavoring and aromatizing agent 10 gr. | | • Coconut as flavoring and aromatizing agent 10 gr. |
| | • Vanilla essence as a flavoring and aromatizing agent 5 ml. | | • Vanilla essence as a flavoring and aromatizing agent 5 ml. |
| | | | • Stevia as sweetening agent 31.2 ml. |
| Hyperproteic Snack: Whey protein powder supplement + spirulina with sugar | | Hyperproteic Snack: Whey protein powder supplement + spirulina sugar free | |
| Ingredients: | | Ingredients: | |
| | • Flour as a binding agent 150 grs | | • Flour as a binding agent 150 grs |
| | • Sugar as sweetening agent 125 grs. | | • Sugar as sweetening agent 125 grs. |
| | • Margarine as lipidic emulsifying agent 100 gr. | | • Margarine as lipidic emulsifying agent 100 gr. |
| | • Whey protein powder supplement with sugar as protein source 60 gr. | | • Whey protein powder supplement with sugar as protein source 60 gr. |
| | • Spirulina as protein source agent 30 gr. | | • Spirulina as protein source agent 30 gr. |
| | • Eggs as binding agent 2 un. | | • Eggs as binding agent 2 un. |
| | • Baking powder as leavening agent 10 gr. | | • Baking powder as leavening agent 10 gr. |
| | • Coconut as flavoring and aromatizing agent 10 gr. | | • Coconut as flavoring and aromatizing agent 10 gr. |
| | • Vanilla essence as a flavoring and aromatizing agent 5 ml. | | • Vanilla essence as a flavoring and aromatizing agent 5 ml. |

This distribution of the caloric molecule in the snack is based on the requirements of a standard patient in HD (Miguel C. Riella; Cristina Martins. (2004). Nutrition and Kidney. Brazil: Editorial Medica Panamericana) since, the contributions of the Snack they should reflect a snack equivalent to 15% of your daily calories. The protein intake must be greater than 15% in order to ensure that it is a hyperproteic food, as this will ensure its protein intake.

The egg white was dehydrated to obtain dehydrated and concentrated ovalbumin. The egg whites were cut with a knife, and thus, achieve homogeneous expansion, then subjected to increasing temperature until reaching 50°C; and it is allowed to dehydrate completely for approximately 12 hours; dries and recovers the dehydrated white from the wood material. The total dehydrated egg white is weighed and ground for later storage.

Mix two eggs plus the protein source shaking with increasing vigor, and then add the sweetening agent, specifically, sugar or liquid stevia according to the variety of snack; add the emulsifying agent selected from margarine and stir until obtaining a homogeneous mixture; add flavoring and flavoring agents, selected from vanilla and coconut essence, and the leavening agent selected from baking powder; add the selected agglomerating agent from sifted flour and stir until a homogeneous mixture is obtained; add the homogeneous mixture into molds with a dispenser; and bake for 10 to 15 minutes until obtaining a product with a desirable color and texture, and later, let it cool for later packaging. A theoretical and proximal bromatological analysis was carried out on the resulting product. The theoretical bromatological analysis compared theoretical contributions against those obtained in the product. See Table 6.

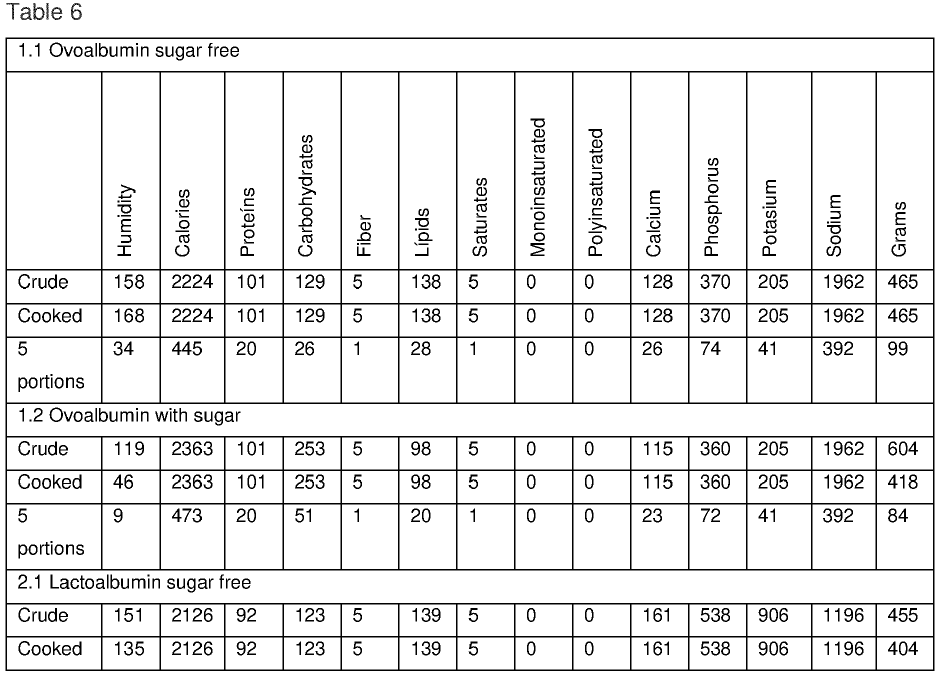

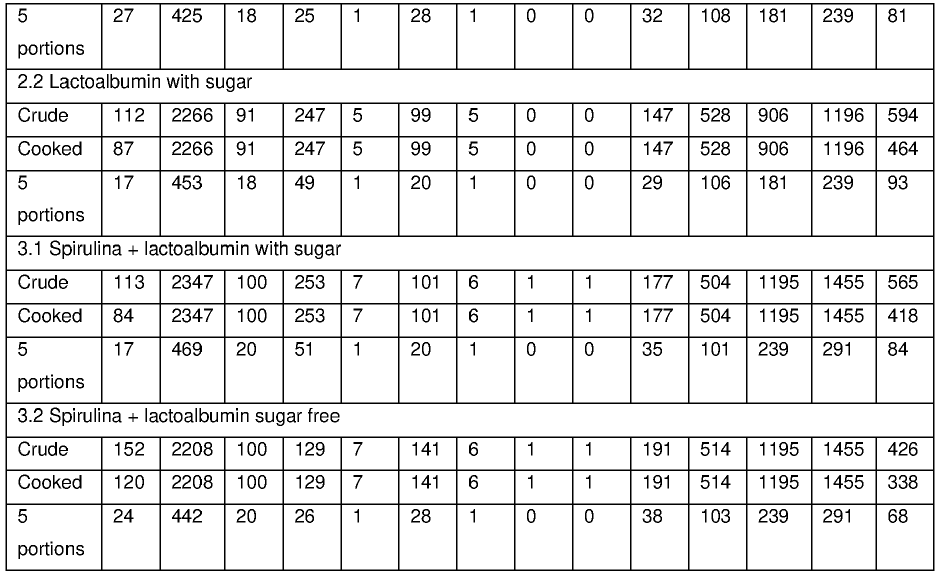

The proximal analysis was carried out to determine the amount of moisture, proteins, lipids, ashes and carbohydrates of the product made. Moisture was determined by establishing the weight ratio of the composition on dry weight and wet basis of the food. And for this, in a dry and tared crystallizer, 2 to 5 grams of sample are weighed exactly, which is heated to 100-130 ° C for 1 to 2 hours, and then cooled in the desiccator and weighed, to then repeat the heating up to constant weight, with a variation of no more than 2 milligrams. The moisture content corresponds to the weight loss during drying, at the temperature used by 10 grams of the original sample, In the determination of proteins by the Kjeldahl method, amino acids, peptides and proteins - important components of food, mainly for their protein synthesis and also because they contribute directly to the flavor of foods and are precursors of the aromatic components, the sample is heated to 100 ^{and} C for 2 hours to constant weight, and takes a digestion tube by adding one gram of copper sulfate and 0.5 grams of potassium sulfate, carefully adding 10 ml of 98% sulfuric acid. Then, 2 ml of hydrogen peroxide is added, turning quickly to a black color, a sign of the destruction of the organic matter product of the dehydration produced by the sulfuric acid. After a time of 2 hours, the sample becomes completely crystalline, which indicates the passage of nitrogen in the form of ammonia. The sample is allowed to cool for one hour, and is then taken to a still with 25 ml of 4% boric acid with 5 drops of mixed indicator, and then between 100-150 ml of 34% sodium hydroxide are added./v. The ammonia is distilled for 5 to 7 minutes to obtain 150 ml of solution, and later all the residues are collected from the tube, to finally carry out a titration with hydrochloric acid. In ovalbumin, it was obtained in 100 grams of powder, 74 grams of protein. While in lactalbumin, 87 grams were obtained and spirulina has 65 grams.

To determine the fat content of a food, according to the characteristics that lipids have of being soluble in solvents, in addition to other components such as phospholipids, sterols, free fatty acids, carotenoid pigments and chlorophyll, information is obtained as crude fat stereo abstract. Exactly 5 grams of sample is weighed, homogenized and dried at 100 ° C for 2 hours, then transferred to a filter paper thimble, placing the thimble in the soxhlet jacket and adding 50 to 60 ml of petroleum ether 6 Ethyl ether, heat until the solvent boils gently. It is extracted for 4 hours, maintaining the volume of the solvent, to subsequently eliminate the remaining solvent in the sample by evaporation, and it is heated for 1 hour at 100 ° C to cool.

For the palatability of the variety of products, a sensory evaluation sheet was prepared. The dishes were labeled by letters with each sample (to start sugar free), the trays were mounted with each sample and card and a glass of water was placed. The judges began to pass five by five, informing them about the procedure and through the boxes, the sugar-free samples mounted on the trays were delivered. The hyperproteic samples with sugar are then delivered.

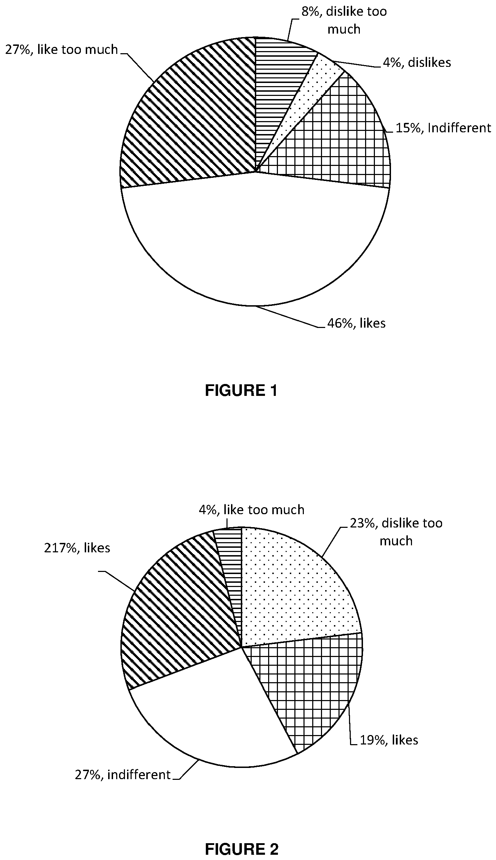

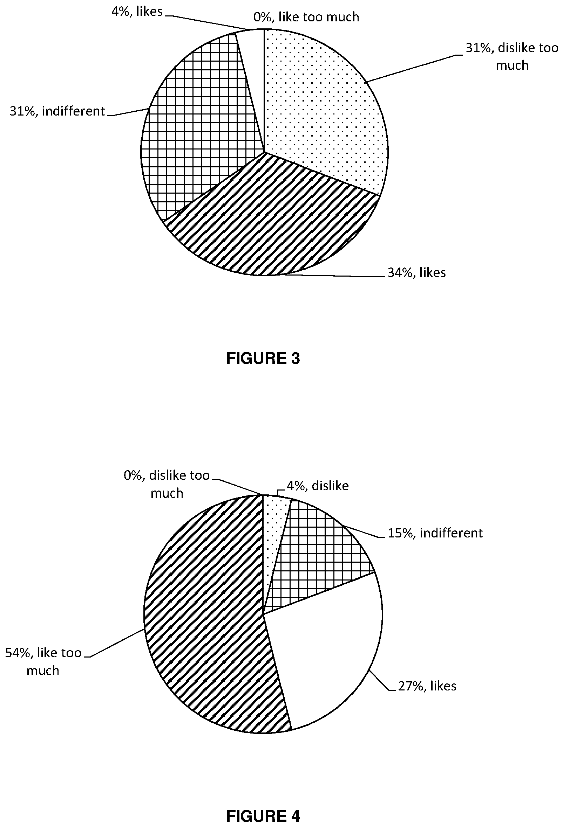

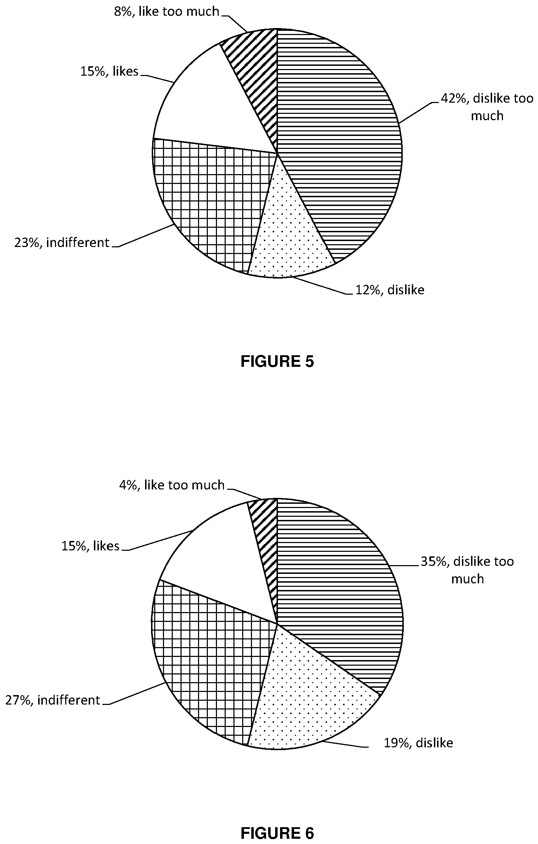

As can be seen from Figure 1, snack 1.1 (ovalbumin sugar free) obtained 46% of the preference of Semi-trained Judges who "Like" the sample, being only 4% those to whom snack 1.1, they "Dislike Too Much". Figure 2 shows the preference according to flavor over the sample of Snack 1 .2 (ovalbumin with sugar), where 27% of the judges indicated that it was "Indifferent" or that they "liked it" while 4% indicated that they" He liked it too much. " Figure 3 shows the preference according to flavor over the sample of Snack 2.1 (Lactalbumin sugar free), where 34% of the judges stated that "It disliked too much" and only 4% of the judges stated that "They like it". Figure 4 shows the preference according to flavor of the sample of Snack 2.2 (Lactalbumin with sugar), where 54% of the judges indicated that they "Like Too Much" and no judge pointed out "I dislike Too much". Figure 5 shows the preference according to taste of sample 3.1 (spirulina + lactalbumin with sugar), where 42% of the judges indicated that they "Dislike Too Much", and only 8% of the judges indicated that they "I like it too much." Figure 6 shows the preference according to flavor to the sample of Snack 3.2 (spirulina + sugar-free lactalbumin), where 35% of the judges indicated that "It Displeases Too Much", and only 4% said "Too Much Dislike".

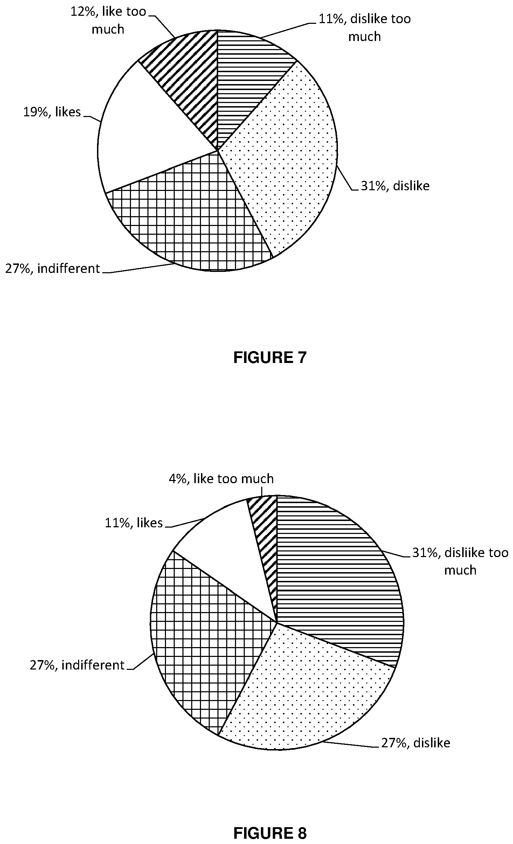

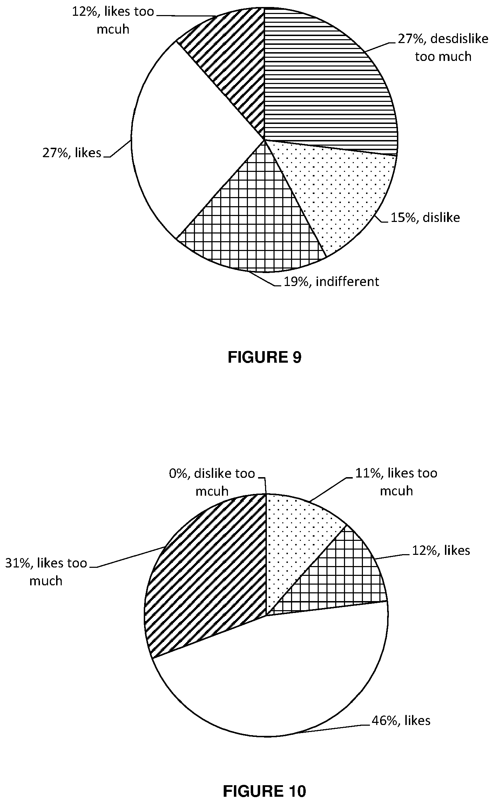

Figures 7 to 9 show the Texture preference of each sample made. Figure 7 shows the preference according to Texture of the sample of Snack 2.2 (lactalbumin with sugar), where 46% of the Semi-trained Judges indicated that they "Like" the texture while 11% indicated that they "Dislike the texture". Figure 8 shows the preference according to Snack texture 3.1 (spirulina + lactoalbumin with sugar), where 38% of the judges indicated that they "Like" the texture while only 4% indicated that they "Like Too Much". Figure 9 shows the preference according to Texture of sample 3.2 (spirulina + lactoalbumin sugar free), where the opinions of the Semi-trained Judges were very varied, because 23% indicated "Too Much Dislike", and also 23% indicated me "Dislike", "Indifferent" and "Pleased" while only 8% indicated "Too Much Dislike". For codes of sensory evaluations, see Table 24.

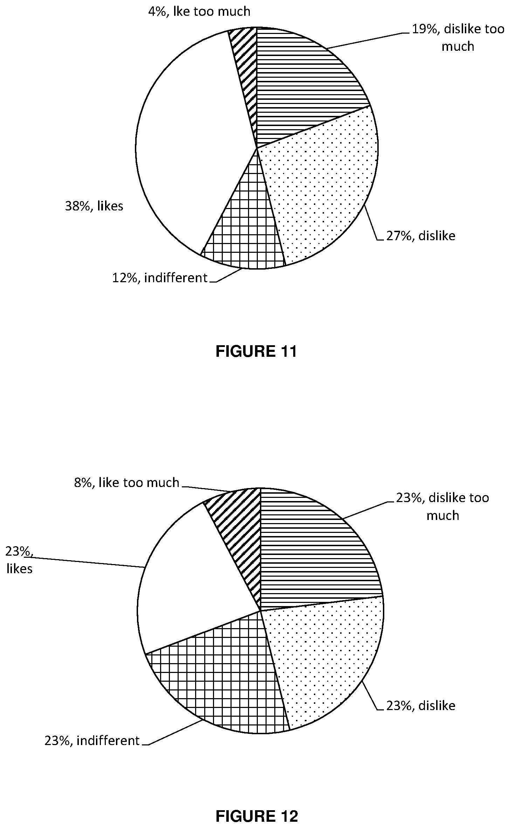

Figures 10 and 11 show the preference of snacks with sugar and sugar free according to Texture and Flavor. In Figure N ° 10 for Snack sugar free, it shows that the snack of Code 1.1 (ovalbumin sugar free) was the most preferred according to Flavor and Texture, and the least preferred was Snack 2.1 (lactalbumin sugar free). In Figure N ° 11, snack with sugar, the most preferred according to Flavor and Texture, was Snack 2.2 (lactalbumin with sugar), and the least preferred was Snack 1.2 (ovalbumin with sugar). For codes of sensory evaluations, see Table 24.

In the bromatological analysis obtained according to Proximal Analysis of the different varieties of hyperproteic Snacks, it is observed that the snacks are not very different in terms of protein intake from the theoretical recipe, but the caloric intake differs. See Table 7.

**Table 7**

| Hyperproteic Snack | Theorical protein grams | Final protein grams | Theorical calories grams | Final calories grams |
|---|---|---|---|---|
| Ovoalbumin sugar free | 20.4 | 18 | 449 | 342 |
| Ovoalbumin with sugar | 23.9 | 17.2 | 562 | 343 |
| Lactoalbumin (whey protein powder supplement) sugar free | 22.6 | 20.6 | 525 | 311 |
| Lactoalbumin (whey protein powder supplement) with sugar | 29.6 | 16.9 | 487 | 427 |
| Spirulina sugar free | 29.5 | 23.9 | 649 | 312 |
| Spirulina with sugar | 23.8 | 18.4 | 558 | 364 |

The whey protein powder supplement with sugar has a contribution of 421 kcal, much higher than the contribution of the other Snacks, since it contains a higher percentage of lipids than the other samples. It should be noted that all the mixes have the same base recipe but for the whey protein powder supplement with sugar a different margarine was used than the other mixes.

Regarding the carbohydrate content, in the dehydrated egg white sample there is a slight difference in the format sugar free compared to that with sugar, and they only have 6% difference compared to the protein powder supplement of serum that contains 22% in the formats with and sugar free.

Regarding humidity, the sample with higher humidity is related to the lack of cooking. Within the Hyperproteic Snacks, the only one that complies with the stipulated humidity is the whey protein powder supplement with sugar, and to improve this parameter it is only necessary to increase the cooking time or temperature. It is known that the more moisture a product has, its duration is shorter, due to the formation of microorganisms, therefore, to produce a hyperproteic product for HD patients, the duration of the product should be at least 3 months. It is also possible to add additional additives such as moisture stabilizing substances, in order to achieve a longer product life. Preferably, the cooking should be carried out at 200°C in 8 min and with homogeneous heat distribution. Optionally, in the preparation of the mixtures, the order in which each ingredient is added can be varied to improve the palatability characteristics of the dough, and obtain a softer product, for example.

Regarding the packaging of this type of Hyperproteic Snack, vacuum packaging is excluded since it significantly deteriorates the presentation of the product.

Tables 7 to 10 show the percentages of adequacy of the theoretical results with respect to the required macronutrients and calories is achieved in most of the Snacks. Proteins and calories are those that fully achieve the proposed objective in all Hyperproteic Snacks, the worst results being those observed in the amount of carbohydrates due to the use of a natural sweetener such as stevia in some samples, and an industrial sweetener. like sugar, in others.

| **Table N°8: Adequacy percent of required proteins compared to a theorical analysis of samples** | | | |
|---|---|---|---|
| Samples | Theorical Prot. | Proteins | % Adequacy |
| 1.1 | 20.40% | 15% | 136 |
| 1.2 | 23.90% | 15% | 159.3 |
| 2.1 | 22.60% | 15% | 150.7 |
| 2.2 | 19.60% | 15% | 130.7 |
| 3.1 | 23.80% | 15% | 158.7 |
| 3.2 | 29.50% | 15% | 196.7 |

| **Table N°9: Adequacy percent of carbohydrate (HC) required compared to a theorical analysis of samples** | | | |
|---|---|---|---|
| Samples | Theorical HC | HC | % Adequacy |
| 1.1 | 26.10% | 50% | 52.2 |
| 1.2 | 60.30% | 50% | 120.6 |

| | | | |
|---|---|---|---|
| 2.1 | 30.40% | 50% | 60.8 |
| 2.2 | 53.20% | 50% | 106.4 |
| 3.1 | 60.30% | 50% | 120.6 |
| 3.2 | 38.00% | 50% | 76 |

| **Table N°10: Adequacy percent of required lipids compared to a theorical analysis of samples** | | | |
|---|---|---|---|
| Samples | Theorical Lip. | Lipids | % Adequacy |
| 1.1 | 27.90% | 25% | 111.6 |
| 1.2 | 23.30% | 25% | 93.2 |
| 2.1 | 34% | 25% | 136.8 |
| 2.2 | 21.20% | 25% | 84.8 |
| 3.1 | 24.00% | 25% | 96 |
| 3.2 | 41.40% | 25% | 165.6 |

| **Table N°11: Adequacy percent of required calories compared to a theorical analysis of samples** | | | |
|---|---|---|---|
| Samples | Theorical Calories | Calories | % Adequacy |
| 1.1 | 449.2 | 350 | 128.3 |
| 1.2 | 562.7 | 350 | 160.8 |
| 2.1 | 525.1 | 350 | 150.0 |
| 2.2 | 487.3 | 350 | 139.2 |
| 3.1 | 558.9 | 350 | 159.7 |
| 3.2 | 649.3 | 350 | 185.5 |

The proximal evaluation allowed to establish the results related to macronutrients and calories of each hyperproteic snack. Table N ° 12 shows the values of the proximal analyzes of each type of Snack.

| **Table N° 11: Results of proximal analysis of different hyperproteic snacks varieties.** | | | | | |
|---|---|---|---|---|---|
| Sample | % Humidity | % Lipids | % proteins | % HC | Calories |
| 1.1 | 24.44 | 10.15 | 18.20 | 44.48 | 342.07 |
| 1.2 | 22.39 | 8.62 | 17.18 | 49.36 | 343.72 |
| 2.1 | 34.22 | 12.84 | 20.67 | 28.26 | 311.27 |
| 2.2 | 14.72 | 17.07 | 16.93 | 50.01 | 421.42 |
| 3.1 | 17.57 | 9.01 | 18.40 | 52.43 | 364.42 |
| 3.2 | 29.67 | 9.07 | 23.97 | 33,71 | 312,40 |

Tables 13 to 16 show the percentage of adequacy of each hyperproteic Snack analyzed proximally with respect to the required protein, carbohydrates, lipids and calories. The protein results are those that fully achieve the objective, as they exceed the 15% protein required in the Snack. With regard to calories, only two Snacks reach the goal of being greater than 350, and the rest of the Snack does not differ excessively from what is proposed. The carbohydrate values are not adjusted correctly in the sugar-free snacks due to the change in the recipe and the lack of nutrient content of stevia.

| **Table N°13 Adequacy percent of required proteins compared to proximal analysis of samples** | | | |
|---|---|---|---|
| Samples | Proximal Prot. | Proteins | % Adequacy |
| 1.1 | 21.30% | 15% | 142 |
| 1.2 | 19.90% | 15% | 132.7 |
| 2.1 | 26.60% | 15% | 177.3 |
| 2.2 | 18.90% | 15% | 126 |
| 3.1 | 20.20% | 15% | 134.7 |
| 3.2 | 30.70% | 15% | 204.7 |

| **Table N°14: Adequacy percent of required carboydrates compared to proximal analysis of samples** | | | |
|---|---|---|---|
| Samples | Proximal HC | HC | % Adequacy |
| 1.1 | 52% | 50% | 104 |
| 1.2 | 57.50% | 50% | 115 |

| | | | |
|---|---|---|---|
| 2.1 | 36.30% | 50% | 72.6 |
| 2.2 | 49.70% | 50% | 99.4 |
| 3.1 | 57.50% | 50% | 115 |
| 3.2 | 43.20% | 50% | 86.4 |

| **Table N°15: Adequacy percent of required lipids compared to proximal analysis of samples** | | | |
|---|---|---|---|
| Samples | Proximal Lip. | Lipids | % Adequacy |
| 1.1 | 26.70% | 25% | 106.8 |
| 1.2 | 22.60% | 25% | 90.4 |
| 2.1 | 37% | 25% | 148.4 |
| 2.2 | 31.40% | 25% | 125.6 |
| 3.1 | 22.30% | 25% | 89.2 |
| 3.2 | 26.10% | 25% | 104.4 |

| **Table N°16: Adequacy percent of required calories compared to proximal analysis of samples** | | | |
|---|---|---|---|
| Samples | Proximal calories | Calories | % Adequacy |
| 1.1 | 342.07 | 350 | 97.7 |
| 1.2 | 343.72 | 350 | 98.2 |
| 2.1 | 311.27 | 350 | 88.9 |
| 2.2 | 421.42 | 350 | 120.4 |
| 3.1 | 364.42 | 350 | 104.1 |
| 3.2 | 312.4 | 350 | 89.3 |

Tables 17 to 21 compare the amount of macronutrients, moisture and calories that each type of Snack presents according to the Theoretical Analysis and the Proximal Analysis. The percentage of adequacy was calculated following the theoretical analysis as a basis, through these tables, it is possible to interpret that the results that are best related are those of proteins and carbohydrates, since the proximal does not differ greatly with the theoretical.

| **Table N°17: Adequacy percent of humidity from samples as theorical analysis and proximal analysis** | | | |
|---|---|---|---|
| Samples | Theorical humidity | Proximal humidity | % Adequacy |
| 1.1 | 33.9 | 24.4 | 72.1 |
| 1.2 | 11.0 | 22.4 | 202.7 |
| 2.1 | 33.2 | 34.2 | 103.0 |
| 2.2 | 18.8 | 14.7 | 78.4 |
| 3.1 | 19.9 | 17.6 | 88.2 |
| 3.2 | 35.4 | 29.7 | 83.8 |

| **Table N°18: Adequacy percent of protein from samples as theorical analysis and proximal analysis** | | | |
|---|---|---|---|
| Samples | Theorical Proteins | Proximal proteins | % Adequacy |
| 1.1 | 20.4 | 18.2 | 89.3 |
| 1.2 | 23.9 | 17.2 | 71.8 |
| 2.1 | 22.6 | 20.7 | 91.4 |
| 2.2 | 19.6 | 16.9 | 86.3 |
| 3.1 | 23.8 | 18.4 | 77.3 |
| 3.2 | 29.5 | 24.0 | 81.2 |

| **Table N°19 Adequacy percent of carbohydrates from samples as theorical analysis and proximal analysis** | | | |
|---|---|---|---|
| Samples | Theorical HC | Proximal HC | % Adequacy |
| 1.1 | 26.1 | 44.5 | 170.5 |
| 1.2 | 60.3 | 49.4 | 81.9 |
| 2.1 | 30.4 | 28.3 | 93.0 |
| 2.2 | 53.2 | 50.0 | 94.1 |
| 3.1 | 60.3 | 52.4 | 86.9 |

| | | | |
|---|---|---|---|
| 3.2 | 38.0 | 33.7 | 88.8 |

| **Table N°20: Adequacy percent of lipids from samples as theorical analysis and proximal analysis** | | | |
|---|---|---|---|
| Samples | Theorical lipids | Proximal lipids | % Adequacy |
| 1.1 | 27.9 | 10.2 | 36.4 |
| 1.2 | 23.3 | 8.6 | 37.0 |
| 2.1 | 34.2 | 12.8 | 37.5 |
| 2.2 | 21.2 | 17.1 | 80.5 |
| 3.1 | 24.0 | 9.0 | 37.5 |
| 3.2 | 41.4 | 9.1 | 21.9 |

| **Table N°21: Adequacy percent of calories from samples as theorical analysis and proximal analysis** | | | |
|---|---|---|---|
| Samples | Theorical calories | Proximal calories | % Adequacy |
| 1.1 | 449.2 | 342.1 | 76.1 |
| 1.2 | 562.7 | 343.7 | 61.1 |
| 2.1 | 525.1 | 311.3 | 59.3 |
| 2.2 | 487.3 | 421.4 | 86.5 |
| 3.1 | 558.9 | 364.4 | 65.2 |
| 3.2 | 649.3 | 312.4 | 48.1 |

The results of the sensory evaluation carried out are shown in tables 22 and 23:

**Table 22: Sensory Analysis Evaluation Responses. Sugar-free snack**

| **Sensorial analysis from samples sugar free** | | | | | | | |
|---|---|---|---|---|---|---|---|
| **Texture text** | | | | **Flavoring Testr** | | | |
| SET | 2.1 | 1.1 | 3.2 | SET | 2.1 | 1.1 | 3.2 |
| 1 | -2 | -2 | -2 | 1 | -1 | -2 | -2 |
| 2 | -1 | -1 | 0 | 2 | -1 | 0 | -2 |
| 3 | -2 | 2 | 0 | 3 | -2 | 2 | 0 |
| 4 | -2 | 0 | -2 | 4 | 0 | 1 | -2 |
| 5 | 2 | 0 | 1 | 5 | 0 | 2 | 1 |
| 6 | 1 | 0 | 2 | 6 | 0 | 1 | 2 |
| 7 | 0 | 1 | -2 | 7 | -1 | 1 | -2 |
| 8 | 1 | -2 | -1 | 8 | -2 | -2 | 1 |
| 9 | 1 | 0 | -1 | 9 | -1 | 1 | 0 |
| 10 | 1 | 1 | -1 | 10 | -2 | 1 | -1 |
| 11 | 1 | -1 | -2 | 11 | -1 | 1 | -2 |
| 12 | 0 | -1 | -2 | 12 | -1 | 0 | -2 |
| 13 | -1 | 2 | 0 | 13 | 0 | 2 | -2 |
| 14 | -1 | 1 | 0 | 14 | -2 | 1 | 0 |
| 15 | 0 | -1 | 1 | 15 | -1 | 0 | 1 |
| 16 | 0 | 0 | -1 | 16 | 0 | 0 | -1 |
| 17 | -2 | -2 | -1 | 17 | -2 | -1 | 1 |
| 18 | 1 | -1 | 1 | 18 | -1 | 1 | 0 |
| 19 | -2 | -1 | 1 | 19 | -2 | 2 | -1 |
| 20 | 1 | 2 | 0 | 20 | 1 | 2 | -2 |
| 21 | 0 | -1 | 1 | 21 | 0 | 1 | 0 |
| 22 | 2 | 1 | 2 | 22 | 0 | 2 | -1 |
| 23 | -2 | 0 | -1 | 23 | -1 | 1 | 0 |
| 24 | -1 | 0 | -2 | 24 | 0 | 1 | -2 |
| 25 | -2 | 1 | 0 | 25 | -2 | 1 | 0 |
| 26 | 2 | -1 | 1 | 26 | -2 | 2 | -1 |

**Table 23: Sensory Analysis Evaluation Responses. Sugar-free snack.**

| **Sensorial analysis from samples with sugar** | | | | | | | | |
|---|---|---|---|---|---|---|---|---|
| **Texture text** | | | | **Flavoring Test** | | | | |
| SET | 2.1 | 1.1 | 3.2 | | SET | 2.1 | 1.1 | 3.2 |
| 1 | -1 | -2 | -2 | | 1 | 0 | -2 | -1 |
| 2 | 1 | -2 | 0 | | 2 | 0 | 0 | 1 |
| 3 | 1 | 0 | 0 | 3 | | 2 | 1 | -2 |
| 4 | 0 | 1 | -2 | 4 | | 2 | 1 | -2 |
| 5 | 2 | 0 | 1 | 5 | | 2 | 0 | 1 |
| 6 | 1 | 0 | -1 | 6 | | 2 | 0 | -2 |
| 7 | 1 | -2 | -1 | 7 | | 1 | 0 | -2 |
| 8 | 1 | -1 | -2 | 8 | | 0 | 1 | -2 |
| 9 | 1 | 0 | -1 | 9 | | 2 | 0 | -1 |
| 10 | 1 | 0 | 1 | 10 | | 1 | 1 | 2 |
| 11 | 2 | 0 | -1 | 11 | | 2 | 1 | -2 |
| 12 | 1 | -1 | -2 | 12 | | 1 | -2 | -1 |
| 13 | 1 | 2 | -1 | 13 | | 2 | 1 | -2 |
| 14 | -1 | 0 | 1 | 14 | | 2 | 1 | 0 |
| 15 | 1 | -1 | 0 | 15 | | 1 | -1 | 0 |
| 16 | 0 | -2 | -1 | 16 | | 0 | -1 | -2 |
| 17 | 1 | -2 | -1 | 17 | | -1 | -2 | -2 |
| 18 | 2 | -1 | 1 | 18 | | 2 | -2 | 0 |
| 19 | 1 | -2 | 2 | 19 | | 1 | -1 | 2 |
| 20 | 2 | -2 | 1 | 20 | | 2 | -2 | 0 |
| 21 | 2 | 1 | -2 | 21 | | 2 | 0 | -2 |
| 22 | 2 | 1 | 1 | 22 | | 2 | 2 | 1 |
| 23 | 0 | -1 | 1 | 23 | | 2 | -1 | 1 |
| 24 | -1 | -2 | 1 | 24 | | 1 | 0 | -2 |
| 25 | 2 | -1 | 1 | 25 | | 1 | -2 | 0 |
| 26 | 2 | -1 | 1 | 26 | | 2 | -1 | 0 |

Tables 21 and 22 correspond to the results observed using the hedonic scale card (see Table 24), where the designated values express dislike or satisfaction with the sample presented.

| **Table N°24: Interpretation of Hyperproteic snack sensorial evaluation results, with sugar and sugar free** | |
|---|---|
| Values | Interpretation |
| -2 | Dislike too much |

| | |
|---|---|
| -1 | Dislike |
| 0 | Indifferent |
| 1 | Like |
| 2 | Like too much |

The previous data shows the preparation of a food product that meets the characteristics of a Hyperproteic Snack with AVB proteins, which were Lactoalbumin, Ovalbumin and Spirulina.

Of which variations were created for each of them, with sugar and sugar free, in order to meet the nutritional needs of patients who are in the process of HD, thus considering diabetics.

Optionally, the food product can be adapted to caloric and protein intake similar to an enteral product. In this case, the snack 1.1 may comprise the following ingredients: Egg, Margarine,

Baking powder, Ovalbumin, Wheat flour, Vanilla essence, Grated coconut, Cinnamon essence, Vegetable coloring and a sweetening agent selected from the group consisting of:
sugar, stevia, tagatose, and sucralose. The selection of sucralose allows the food product to be useful for DM patients attending HD. In this case, the food product has the constitution shown in table 25 and adequate macronutrients to the contributions of an enteral product (Table 26).

**Table N ° 25: Ingredients and grammages of the recipe for the functional hyperproteic snack.**

| Ingredient | Grams |
|---|---|
| Wheat flour as binding agent | 45 |
| Margarine as emulsifying agent | 25 |
| Eggs as binding agent | 30 |
| Ovoalbumin as protein | 10 |
| Vanilla essence as aromatizing and flavoring agent | 6 |
| Grated Coconut as aromatizing and flavoring agent | 6 |
| Cinnamon essence as flavoring and aromatizing agent | 0.5 |
| Sucralose as sweetening agent | 2 |
| Baking powder as leavening agent | 1 |

Optionally, the food product has a ratio of binding agent to emulsifying agent to protein binding agent to flavoring and flavoring agents to sweetening agent to leavening agent of 45: 25: 30: 10: 12.5: 2: 1.

**Table No. 26 : Nutritional labeling of functional hyperproteic snack**

| NUTRITIONAL INFORMATION | | |
|---|---|---|
| Portion: 2 muffins (108 g) | | |
| Container Portion: 1 portion | | |
| | 100 g | 1 Portion |
| Energy (Kcal) | 376.5 | 406.63 |
| Proteins (gr) | 16.18 | 17.48 |
| Total Fatl (gr) | 18.83 | 20.34 |
| Carbhydrate (gr) | 35.25 | 38.07 |
| Sodium (mg) | 363.44 | 392.52 |
| Potasium (mg) | 79.12 | 85.45 |
| Phosphorus (mg) | 22.94 | 24.78 |
| Humidity | 34 | 37 |
| Ingredients: Wheat flour, margarine, eggs, ovalbumin, grated coconut, vanilla essence, cinnamon essence, sucralose, vegetal colorant. | | |
| Duration: 3-4 days after prepared | | |

**Table 27 shows a comparison of the results of the Weende proximal analysis, the results of macronutrients and moisture, obtained from a theoretical nutritional labeling.**

| | Humidity | Proteins | Lipids | CHO |
|---|---|---|---|---|
| Proximal analysis (gr) | 36.2 | 14.3 | 15.7 | 30.04 |
| Theorical nutritional Labelling (gr) | 37 | 16.8 | 18.83 | 35.26 |
| % difference¹ | 2.16 | 13.94 | 19.94 | 17.38 |

| | | | | |
|---|---|---|---|---|
| ¹ % difference = (Higher sample value - Lower sample value)/Lower sample value x 100 In relation to the traditional enteral products compared to the functional hyperproteic snack, it is observed that the traditional enteral formulas have 332% higher humidity compared to the hyperproteic snack. | | | | |

On the other hand, the proximal analysis calories are 318.2 while the theoretical calories are 376.5, the difference being 18.32%.

## Claims

1. Snack-type food product with high hypoprotein content comprising proteins of high biological value (BVS), the main macronutrients being protein, carbohydrates, carbohydrates and fats, in addition to at least one or more sweetening agents, leavening agents, binding agents, binding agents, flavoring agents, flavoring agents, and optionally preserving agents, confectionery coating agents.

2. The product of claim 1 being proteins of animal or vegetable origin.

3. The product of claim 2 being said proteins of animal origin selected from lactoalbumin or ovalbumin.

4. The product of claim 3 being said protein of animal origin, lactoalbumin.

5. The product of claim 2 being said protein of animal origin, ovalbumin.

6. The product of claim 1 being said binding agent, flour.

7. The product of claim 1 being said lipid emulsifying agent, margarine.

8. The product of claim 1 being said binding agent, dehydrated egg white, egg or both.

9. The product of claim 1 being said raising agent, baking powder.

10. The product of claim 1 being said flavoring and flavoring agent, coconut or vanilla essence.

11. The product of claim 1 being said sweetening agent selected from sugar or stevia.

12. The product of claim 1 being the ratio of binding agent to lipid emulsifying agent to binding agent to leavening agent to sweetening agent is 150: 150: 90: 10: 10: 31.2.

13. Use of the product of claim 1 being useful for patients undergoing hemodialysis treatment.

14. The product of any of claims 1 to 12, comprising the following ratio of components: Wheat flour as binding agent is Margarine as emulsifying agent is Egg as binding agent is ovalbumin as protein is vanilla essence as flavoring and flavoring agent is coconut grated as a flavoring and flavoring agent is to cinnamon essence as a flavoring and flavoring agent is to sucralose as a sweetening agent is to baking powder as a leavening agent as 45: 25: 30: 10: 6: 0.5: 2: 1.

15. A method for preparing a snack-type food product with a high hypoprotein content comprising dehydrating egg white to obtain dehydrated and concentrated ovalbumin; cut with a knife, the egg whites, and thus, achieve homogeneous expansion, then subject to increasing temperature until reaching 50 ° C; and allow to dehydrate completely; dry and recover the dehydrated white and grind it; mix two eggs plus the dehydrated and ground white, shaking with increasing vigor, then add: the sweetening agent, preferably sugar or liquid stevia; the emulsifying agent, preferably margarine; and stir until a homogeneous mixture is obtained; add flavoring and flavoring agents, preferably vanilla and coconut essence, and the leavening agent, preferably baking powder; add the agglomerating agent, preferably sifted flour and stir until a homogeneous mixture is obtained; add the homogeneous mixture into molds with a dispenser; and bake for 10 to 15 minutes until obtaining a product of desirable color and texture, and later, let it cool for later packaging.
